# EUROPEAN PATENT SPECIFICATION

(11) **EP 4 523 189 B1**
(45) Date of publication and mention of the grant of the patent: **31.12.2025**
(21) Application number: 23724842.2
(22) Date of filing: 10.05.2023
(51) Int. Cl.: G07F 11/24, G07F 11/44, G07F 17/00, G01V 8/00

(54) **SENSOR SYSTEM, ASSEMBLY, METHOD AND COMPUTER PROGRAM PRODUCT FOR DETECTING EVENTS IN AN AUTOMATIC DISPENSING PROCESS OF DISCRETE MEDICAMENTS**
SENSORSYSTEM, ANORDNUNG, VERFAHREN UND COMPUTERPROGRAMMPRODUKT ZUR DETEKTION VON EREIGNISSEN IN EINEM AUTOMATISCHEN AUSGABEPROZESS DISKRETER MEDIKAMENTE
SYSTÈME DE CAPTEUR, ENSEMBLE, PROCÉDÉ ET PRODUIT PROGRAMME D'ORDINATEUR POUR DÉTECTER DES ÉVÉNEMENTS DANS UN PROCESSUS DE DISTRIBUTION AUTOMATIQUE DE MÉDICAMENTS DISCRETS

(30) Priority: 11.05.2022 NL 2031831
(43) Date of publication of application: 19.03.2025
(73) Proprietor: VMI Holland B.V., 8161 RK Epe (NL)
(72) Inventor: BRAKKEE, Martinus Johannes Donatus, 8161 RK Epe (NL); VAN ROON, Peter, 8161 RK Epe (NL); WIJNIA, Aalf, 8161 RK Epe (NL)
(74) Representative: Nederlandsch Octrooibureau
(86) International application number: PCT/EP2023/062458
(87) International publication number: WO 2023/217882

(56) References cited:
- EP-A1- 3 815 668
- KR-A- 20160 012 594
- US-A1- 2012 029 692

## Description

### BACKGROUND

The invention relates to a sensor system, an assembly, a method and a computer program product for detecting events in an automatic dispensing process of discrete medicaments.

US 10,173,830 B1 discloses a system which accommodates a series of medication dispensing containers, also known as 'feeder units' or 'canisters', for selectively dispensing an amount of said solid medications from one or more medication dispensing containers and for packing said dispensed amount. Each medication dispensing container holds an amount of solid substances specific to that respective medication dispensing container. Hence, together, the medication dispensing containers can dispense a wide variety of solid substances.

The medication dispensing container comprises an arranging body with a plurality of passageways, wherein each passageway is precisely dimensioned for accommodating said solid medications in a single row.

In use, the medication dispensing containers are docked to a holding device. When the medication dispensing container is correctly docked to the holding device, positioning columns of said holding device reach through detector openings into the medication dispensing container. Light sources and light detectors associated with said holding device align with the medication dispensing container and form detection pairs to detect the passage of medicaments through the passageways and through the outlet. EP3815668 A1 is another example of a medication dispensing device with a sensor system.

### SUMMARY

A disadvantage of the known system is that the medication dispensing container and/or the holding device become polluted over time with dust or particles. The pollution can interfere with the transmission of light beams from the light sources towards the respective light detectors. If the medication dispensing container and the holding device are not cleaned in due time, the detection pairs may generate false detection result or will not be able to detect anything at all.

Additionally, the characteristics of the light sources and the light detectors may deviate slightly from their respective factory specifications or the tolerances in the factory specifications are not small enough for the intended application. This makes it difficult for the detection pairs to work together properly.

Moreover, the light source may be subject to light output degradation due to aging. At some point, the light output may be insufficient to properly detect medicaments passing the detection pair.

Finally, when pollution or damage, for example scratches, prevents a proper detection of medicaments, the pollution needs to be removed. The medication dispensing containers can be removed relatively easily for cleaning or repairs and cause minimal downtime. In contrast, the holding devices may be considerably more difficult to remove for cleaning. However, it is unknown to the pharmacist which one of the medication dispensing container and the holding device is polluted, or if both are polluted.

It is an object of the present invention to provide a sensor system, an assembly, a method and a computer program product for detecting events in an automatic dispensing process for dispensing discrete medicaments, wherein the reliability of the detection can be improved and/or pollution can be removed more effectively.

The invention provides a sensor system for detecting events in an automatic dispensing process for dispensing discrete medicaments, wherein the sensor system comprises a light emitter for generating light output, a light receiver for converting light input into one or more light input signals indicative of light input values and a control unit that is operationally connected to said light emitter and said light receiver, wherein the light emitter forms a detection pair with the light receiver, wherein the control unit is configured for receiving the one or more light input signals from the light receiver, wherein the control unit is further configured for operating in a dynamic mode in which the light output of the light emitter is adjusted in response to the light input values.

The sensor system can be used in an assembly for detecting dispensing events of a feeder unit that is being docked to a feeder dock, as will be described in more detail later in this summary.

In the context of the present invention, the 'detection pair' is not limited to a combination of a single light emitter and a single light receiver. Instead, the light emitter may be part of or comprise an array of a plurality of light emitters, for example to form a light curtain. Similarly, the light receiver may be part of or comprise an array of a plurality of light receivers. As such, the array of light emitters and/or light receivers may be deemed the light emitter and light receiver of the detection pair.

By adjusting the light output, any negative effects of pollution between the light emitter and the light receiver of the detection pair, age degradation of the light emitter or slight variations in the characteristics of the light emitter and the light receiver of the detection pair can be at least partially compensated. In particular, the light output may be controlled such that the light input received by the respective light receiver is or remains within an optimal operating range or window for said light receiver. Hence, the detection pair can continue to detect events of in the automatic dispensing process reliably.

The control unit, in the dynamic mode, is configured for increasing the light output of the light emitter when the light input values decrease over time. Hence, even when the light beam between the light emitter and the light receiver of the detection pair is hindered by pollution, the light receiver can still receive the increased light output in order to reliably detect the events. Moreover, the light output of the light emitter may be used as an indicator of how polluted the sensor system, the feeder unit and/or the feeder dock are; e.g. the higher the light output, the higher the pollution. The increase in light output may be compared to a fixed value or a previously determined reference value, for example a light output level that has been determined during a prior calibration of the sensor system.

In the dynamic mode, the control unit is configured for increasing the light output of the light emitter when the light input values drop below an input threshold. The input threshold may be chosen at a level which is within the optimal operating range or window of the light receiver, i.e. the range in which the light receiver is most sensitive to light.

Preferably, the input threshold is a predetermined input threshold value stored in a memory. The input threshold value may be chosen by the pharmacist or another operator, or it may be based on factory specifications of the light receiver.

The control unit is configured for switching between the dynamic mode and a calibration mode, wherein the input threshold is a calibration value that is determined based on one or more of the light input values during the calibration mode. Hence, the input threshold may be adjusted to best suit the conditions as measured during the calibration mode, for example taking into account variations in the light output, such as age degradation, and/or any pollution between the light emitter and the light receiver of the detection pair.

In a further embodiment the input threshold is equal to or less than sixty percent of a maximum light input signal of the light receiver pair. By choosing the input threshold below sixty percent, it can be ensured that the light receiver is properly triggered and/or activated by the light input that is received.

In another embodiment the control unit, in the dynamic mode, is configured for storing the light input values continuously or at a regular interval. By storing the light input values continuously, any decrease in the light input values can be detected early and/or accurately. Alternatively, storing the light input values at regular intervals may reduce the data consumed by storing the light input values. This can be, for example, at regularly scheduled times, after a certain period of use or other intervals.

In another embodiment the control unit, in the dynamic mode, is configured for increasing the light output of the light emitter up to an output threshold. Preferably, the output threshold is equal to or less than ninety percent of a maximum output of the light emitter. When said output threshold has been reached, it can be assumed that the sensor system, the feeder unit and/or the feeder dock have become too polluted to continue dispensing and detecting in a reliable manner.

The invention provides an assembly of the sensor system according to any one of the previously discussed embodiments and a feeder dock for docking a feeder unit, wherein the light emitter and/or the light receiver are provided in, on or at the feeder dock.

The feeder unit comprises a dispensing mechanism for dispensing the discrete medicaments one-by-one, wherein the feeder dock comprises a dispensing drive that is arranged for engaging with the dispensing mechanism when said feeder unit is received at the feeder dock, wherein the control unit is operationally connected to the dispensing drive to operate the dispensing mechanism at dispensing intervals, wherein the control unit, in the dynamic mode, is configured for storing the light input values for the one or more light input signals that are received between the dispensing intervals. Hence, the light input values which are stored correspond to a light beam between the light emitter and the light receiver of the detection pair which was uninterrupted, or which at least was not interrupted or blocked by a discrete medicament at the time.

The feeder unit has an at least partially transparent housing, wherein the light emitter and the light receiver are positioned such that, when the feeder unit is received in the feeder dock, a light beam travelling between the light emitter and the light receiver travels through a transparent section of the at least partially transparent housing at least once. The transparent section can become polluted by dust or particles of the discrete medicaments that pass through and out of the feeder unit. By positioning the detection pair in such a way that the light beam passes through the transparent section, allows for the sensor system to not only detect the discrete medicaments, but also get an indication of pollution of the feeder unit.

The control unit is configured for switching between the dynamic mode and a calibration mode, wherein the control unit, in the calibration mode, is configured for storing one or more of the light input values as a calibration value when the feeder unit is absent from the feeder dock. When the feeder unit is absent from the feeder dock, the light beam can be transmitted from the light emitter to the light receiver substantially uninterrupted. Hence, the light input value that is measured during the calibration mode can be used as an indication of what the light receiver should measure as a light input value when the transparent section of the feeder unit is clean.

The control unit is configured for switching between the dynamic mode and a test mode, wherein the control unit, in the test mode, is configured for storing one or more of the light input values in a first situation in which the feeder unit is docked at the feeder dock and for storing one or more of the light input values in a second situation in which the feeder unit is removed from the feeder dock. The light input values from the different situations can be compared, automatically or by the operator, to assess the condition of the feeder dock and the feeder unit. In particular, one can establish whether removing the feeder unit has a positive influence on the light input values, which could be an indication of the feeder unit being polluted. When the light input values in both situations are more or less the same, any decrease in the light input values during the dynamic mode can be contributed to pollution of the feeder dock.

The control unit is further configured for comparing the one or more light input values stored in the first situation with the one or more light input values stored in the second situation. Hence, the assessment discussed above can be made automatically.

In another embodiment the control unit is further configured for determining, based on the comparison, if one of the following conditions is true:
a) one of the first situation and the second situation results in the light input values being different than in the other of the first situation and the second situation; or
b) the light input values are the same or within a predetermined tolerance of each other for the first situation and the second situation. Again, the assessment discussed above can be made automatically.

In another embodiment the assembly comprises an alerting device for notifying an operator, wherein the control unit is operationally connected to the alerting device for sending a first notification to the operator via said alerting device when condition a) is true and for sending a second notification to the operator via said alerting device when condition b) is true. The operator can subsequently take appropriate action, for example cleaning one or both of the feeder dock and the feeder unit. When only the feeder unit is polluted, the feeder unit can be removed from the feeder dock without the need to inspect the feeder dock. Hence, the dispensing operation can be continued with minimal downtime, for example by docking a replacement feeder unit to the feeder dock while the removed feeder unit is being cleaned.

The control unit is configured for switching from the dynamic mode to the test mode when the light output of the light emitter is equal to or higher than an output threshold. Preferably, the output threshold is less than ninety percent of a maximum output of the light emitter. When said output threshold has been reached, it can be assumed that the sensor system, the feeder unit and/or the feeder dock have become too polluted to continue dispensing and detecting in a reliable manner. The test mode can then be triggered automatically.

It is noted that the test mode can also be executed independently from the dynamic mode, for example merely to do a check on the pollution of the feeder dock and/or the feeder unit, and can thus be made of divisional applications not requiring the control unit operating in a dynamic mode.

According to another aspect, the invention provides a method for detecting events in an automatic dispensing process for dispensing discrete medicaments using the assembly previously described, wherein the method comprises the steps of:
- emitting light output from a light emitter of a detection pair;
- receiving light input by a light receiver of the detection pair and converting light input into one or more light input signals indicative of light input values;
- monitoring the light input values; and
- adjusting the light output of the light emitter in response to the light input values.

The method relates to the practical implementation of the sensor system according to the first aspect of the invention and/or the assembly according to the second aspect of the invention and thus has the same technical advantages, which will not be repeated hereafter.

In one embodiment the light output of the light emitter is increased when the light input values decrease over time.

In one embodiment the light output of the light emitter is increased when the light input values drop below an input threshold.

Preferably, the input threshold is a predetermined input threshold value.

Alternatively, the input threshold is a calibration value that is determined based on one or more of the light input values during a calibration mode.

In another embodiment the calibration value is determined when a light beam between the light emitter and the light receiver is unobstructed.

In another embodiment the input threshold is equal to or less than sixty percent of a maximum light input signal of the light receiver.

In another embodiment the light input values are monitored continuously or at a regular interval.

In another embodiment the light output of the light emitter is increased up to an output threshold.

In another embodiment the output threshold is less than ninety percent of a maximum output of the light emitter.

In another embodiment the method further comprises the step of:
- dispensing the discrete medicaments one-by-one at dispensing intervals, wherein the light input values are monitored for the one or more light input signals that are received between the dispensing intervals.

In another embodiment the method further comprises the steps of:
;
- in a first situation docking the feeder unit at the feeder dock and monitoring one or more of the light input values when the feeder unit is docked at the feeder dock;
- in a second situation removing the feeder unit from the feeder dock and monitoring one or more of the light input values when the feeder unit is removed from the feeder dock; and
- comparing the one or more light input values monitored in the first situation with the one or more light input values monitored in the second situation.

It is again submitted that the methods according to the embodiment described above can be applied independently of the step of increasing the light output when the light input values decrease over time, for example for the purpose of merely doing a check on the pollution of the feeder dock and/or the feeder unit.

In another embodiment the method further comprises the step of:
- determining, based on the comparison, if one of the following conditions is true:
   a) one of the first situation and the second situation results in the light input values being different than in the other of the first situation and the second situation; or
   b) the light input values are the same or within a predetermined tolerance of each other for the first situation and the second situation, wherein the method further comprises the steps of:
      - sending a first notification to an operator when condition a) is true; and
      - sending a second notification to an operator when condition b) is true.

In another embodiment the monitoring of the light input values in the first situation and the second situation and the comparison thereof are performed when the light output of the light emitter is equal to or higher than an output threshold.

Preferably, the output threshold is less than ninety percent of a maximum output of the light emitter.

According to another aspect, the invention provides a computer program product comprising a non-transitory computer-readable medium holding instructions that, when executed by a processor, cause the assembly previously described to perform the steps of the method previously described.

The various aspects and features described and shown in the specification can be applied, individually, wherever possible. These individual aspects, in particular the aspects and features described in the attached dependent claims, can be made subject of divisional patent applications.

### BRIEF DESCRIPTION OF THE DRAWINGS

The invention will be elucidated on the basis of an exemplary embodiment shown in the attached schematic drawings, in which:
figure 1 shows a side view of an assembly of a sensor system for detecting events in an automatic dispensing process of a feeder unit and a feeder dock for docking said feeder unit; and
figure 2 shows side view of the assembly of figure 1 with the feeder unit received at said feeder dock;
figure 3 shows a flow chart of a method for operating the sensor system of figures 1 and 2 in a dynamic mode; and
figure 4 shows a flow chart of a method for when switching between the dynamic mode of figure 3 and a test mode.

### DETAILED DESCRIPTION OF THE INVENTION

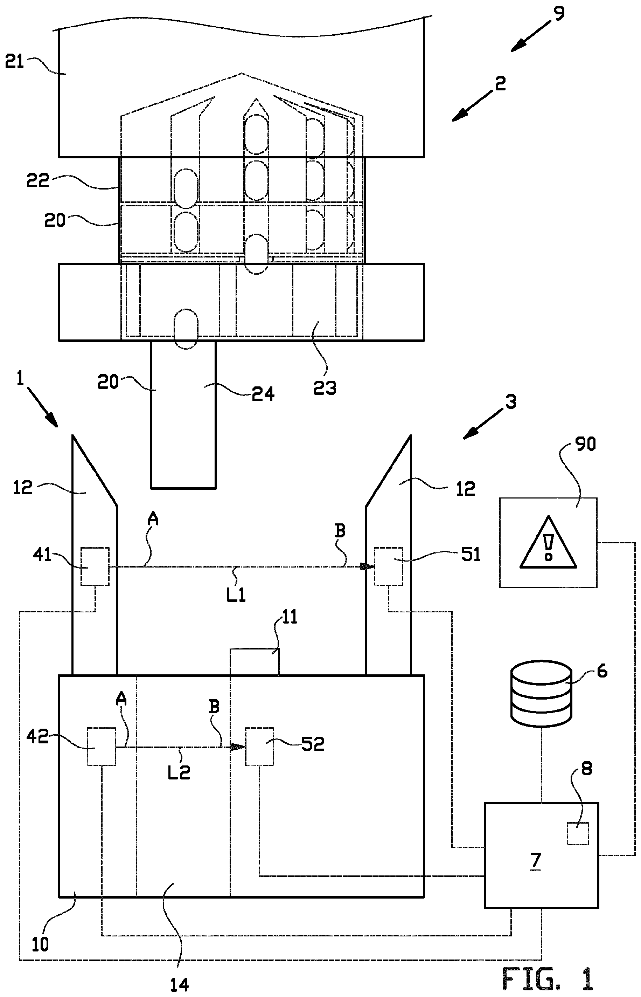

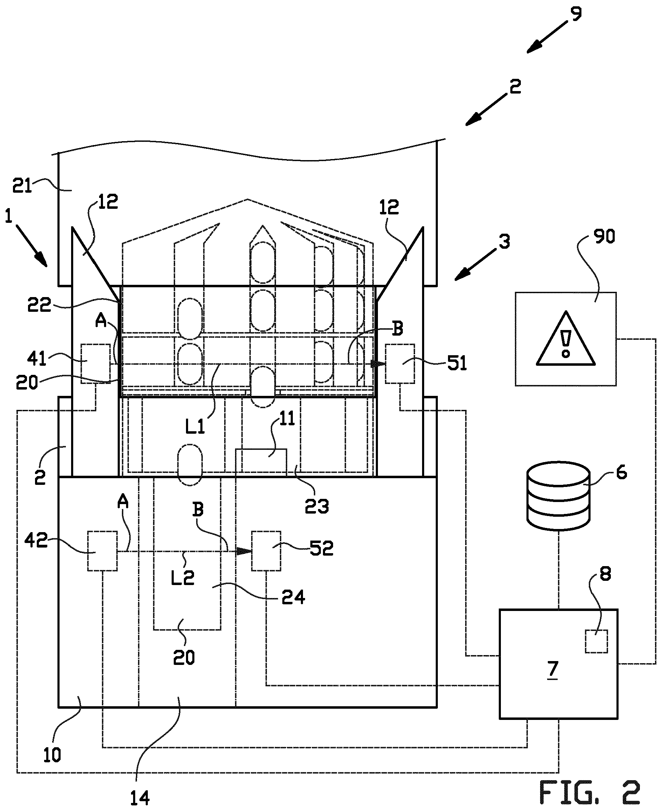

Figures 1 and 2 shows an assembly 9 of a feeder dock 1 and a feeder unit 2 for use in an automatic dispensing process of or for dispensing discrete medicaments, in particular solid medicaments such as pills, tablets or capsules. The medicaments are 'discrete' or 'distinct' in the sense that they can be dispensed one-by-one or in dose-units.

The assembly 9 may be part of an automatic dispensing system, in particular the system according to US 10,173,830 B1, having a plurality of feeder docks and feeder units, each with their own medicaments, to dispense and pack a wide variety of medicaments.

The feeder unit 2 may also be referred to as a 'medication dispensing container' or a 'canister'.

As shown in figure 1, the feeder dock 1 comprises a base 10 for receiving or docking the feeder unit 2, a dispensing drive 11 for engaging with and driving the feeder unit 2 and a plurality of positioning members 12, in particular positioning columns, for aligning and/or positioning the feeder unit 2 relative to the base 10 when docking. The feeder dock 1 further comprises an outlet channel 14. In the situation as shown in figure 1, the feeder unit 2 is removed from or not yet placed onto the feeder dock **1.** Figure 2 shows the situation when the feeder unit 2 has been docked to the feeder dock 1.

The feeder unit 2 comprises a supply chamber 21 for holding the discrete medicaments, and an outlet 24 for dispensing the discrete medicaments from the feeder unit 2 and a dispensing section 22 between the supply chamber 21 and the outlet 24 for feeding the discrete medicaments from the supply chamber 21 to the outlet 24. In particular, the dispensing section 22 comprises a dispensing mechanism 23, similar to the one disclosed in US 10,173,830 B1, with one or more rotatable bodies with passageways for arranging the discrete medicaments in a single row, singulating the discrete medicaments and dispensing them one-by-one from respective waiting chambers into the outlet 24. When the feeder unit 2 is docked to the feeder dock 1, as shown in figure 2, the outlet 24 is aligned with and/or extends into the outlet channel 14 of the feeder dock 1.

The feeder unit 2 has an at least partially transparent housing 20. For example, the feeder unit 2 can have transparent sections of the housing 20 at the dispensing section 22 and/or the outlet 24.

The assembly 9 further comprises a sensor system 3 for detecting events in the automatic dispensing process. In this example, the sensor system 3 comprises a first light emitter 41 and a second light emitter 42 for generating a light output A. In particular, the first light emitter 41 and the second light emitter 42 generate a first light beam L1 and a second light beam L2, respectively. The light output A may be in the visible light spectrum or the invisible light spectrum. The light output A may for example be infrared or a laser light.

In this example, the light emitters 41, 42 are light emitting diodes (LED's). The light emitters 41, 42 may be part of a light curtain formed by several side-by-side light emitters, or the light emitters 41, 42 may be in separate detection areas of the sensor system 3, the feeder dock 1 and/or the feeder unit 2.

The sensor system 3 further comprises a first light receiver 51 and a second light receiver 52, for example in the form of photo cells. Each light emitter 41, 42 forms a detection pair 41, 51; 42, 52 with a respective one of the light receivers 51, 52. The light receivers 51, 52 are arranged for receiving the light output A emitted by the respective light emitters 41, 42 as a light input B and for converting said light input B into one or more input signals C, for example a single continuous input signal C that may vary in magnitude or a plurality of input signals C taken at certain intervals. The input signals are analogue, meaning that they represent a light input value D1, D2, ..., Dn indicative of the strength and/or magnitude of the light input A that is being sensed, measured or detected. The light receivers 51, 52 may be located opposite to the light emitters 41, 42, as shown in figure 1, at the same side as the light emitters 41, 42, in case reflectors (not shown) are being used.

The light emitters 41, 42 and the light receivers 51, 52 are arranged at, on or in the feeder dock 1. They are positioned in such a way that the light beams L1, L2 travelling between the light emitter 41, 42 and the light receiver 51, 52 of the detection pairs 41, 51; 42, 52 travel through a respective one of the transparent sections of the at least partially transparent housing 20 at least once. In this example, the first light emitter 41 and the first light receiver 51 have been positioned in the positioning members 12 on opposite sides of the dispensing section 22. The second light emitter 42 and the second light receiver 52 have been positioned in the base 10 on opposite sides of the outlet 24.

Alternatively, one or more of the light emitters 41, 42 and/or the light receivers 51, 52 may be positioned in the base 10, the positioning members 12 or in dedicated protrusions extending along and/or at least partially into the feeder unit 2 from the feeder dock 1. In a further alternative embodiment, the one or more of the light emitters 41, 42 and/or the light receivers 51, 52 may be arranged at, on or in the feeder unit 2, either in a standalone manner or connected to the feeder dock 1 or another component when the feeder unit 2 is docked to said feeder dock 1.

The sensor system 3 further comprises a memory 6 for storing the light input values D1, D2, ..., Dn and a control unit 7 for processing the light input signals C or the light input values D1, D2, ..., Dn, and for sending a light output value E to the light emitters 41, 42 in response to the light input signals C or the light input values D1, D2, ..., Dn in a manner that will be discussed hereafter in more detail. The memory 6, in this example, is a computer-readable medium which is non-transitory or tangible, e.g. a physical data carrier such as a hard-drive, a USB-drive, a RAM memory or the like. A computer program product in the form of computer-readable instructions may be loaded onto the memory 6 and/or into the control unit 7. The memory 6 can further be used to store the light input values D1, D2, ..., Dn in a database like structure.

The control unit 7 is operationally connected to said one or more light emitters 41, 42, said one or more light receivers 51, 52 and said memory 6. The control unit 7 comprises a processor 8 for processing and/or carrying out computer readable instructions, for example instructions the memory 6 that cause the control unit 7 to carry out steps of a method according to the present invention.

The assembly 9 as a whole, or the sensor system 3 specifically, may further include an alerting device 90 for alerting an operator, for example a pharmacist, of a particular condition and/or actions to be taken. The alerting device 90 may be formed by a human machine interface, for example a display or a screen, or by one or more indicators, for example light and/or audio indicators. The alerting device 90 is used to indicate the state of the feeder dock 1 and/or the feeder unit 2, for example whether the feeder dock 1 and/or the feeder unit 2 are clean or polluted.

A method for detecting events in the automatic dispensing process for dispensing discrete medicaments will now be briefly elucidated with reference to figures 1-4.

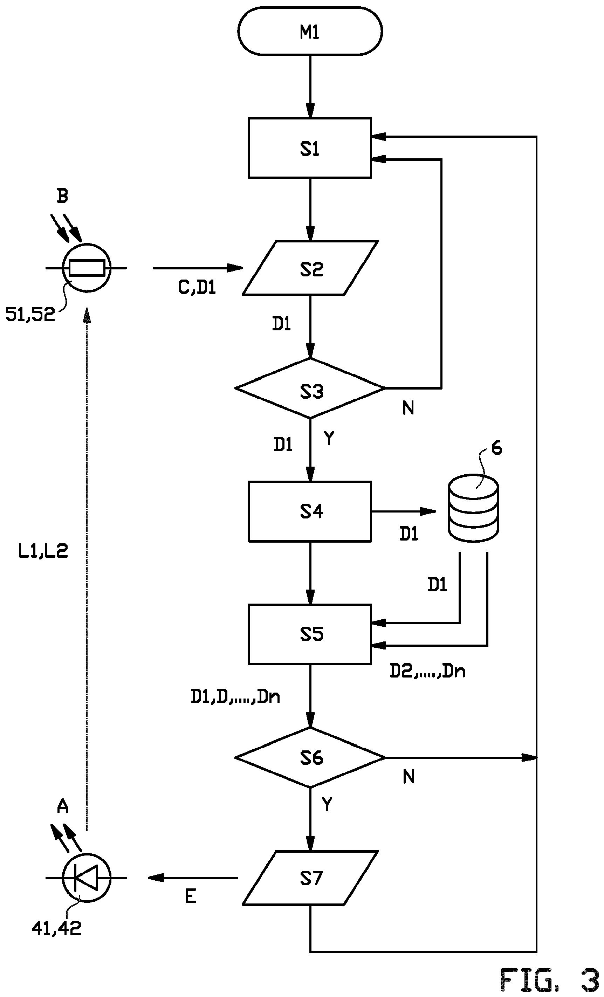

Figure 3 shows the steps of the method when the control unit 7 is operating in a dynamic mode M1.

At the start of the dynamic mode (step S1) a light input signal C or a light input value D1 is received from one of the light receivers 51, 52 (step S2) and processed by the processor 8 of the control unit 7. The control unit 7 is subsequently configured to check whether the light input value D1 has been received between dispensing intervals (Step S3). If the answer is 'yes' (Y) then the light input value D1 is stored in the memory 6 (step S4). If the answer is 'no' (N) then the method returns to the start (step S1).

The light input value D1 that is stored in the previous step (step S4) will hereafter be referred to as the 'last' light input value D1. Light input values D2, ..., Dn stored during previous cycles of the method are referred to hereafter as 'previously stored' light input values D2, ..., Dn.

In the next step of the method (step S5), the control unit 7 is instructed to compare the last light input value D1 with the previously stored light input value(s) D2, ..., Dn. In particular, the control unit 7 checks (steps S6) whether the last light input value D1 has decreased compared to the previously stored light input value D2, ..., Dn. When the answer is 'yes' (Y), the control unit 7 sends an instruction or a light output value E to the respective light emitter 41, 42 to increase the light output A (step S7). If the answer is 'no' (N), the last light input value D1 is stored in the memory 6 as one of the previously stored light input values D2, ..., Dn and the steps S1 to S7 are repeated for the next 'last' light input value D1.

The control unit 7 may be configured to increase the light output value E or the light output A of the light emitter 41, 42 for any decrease in the last light input value D1 compared to the previously stored light input values D2, ..., Dn. Alternatively, the control unit 7 may be configured for increasing the light output value E or the light output A of the light emitter 41, 42 only when the last light input values D1 drops below an input threshold. Said input threshold may be a predetermined input threshold value stored in the memory 6. It may for example be based on a percentage of the maximum light input signal of the light receiver 51, 52 or at the bottom end of an optimum working range of said the light receiver 51, 52. Such a value may for example be found in the factory specifications of the light receivers 51, 52. In this example, the input threshold is chosen to be equal to or less than sixty percent of a maximum light input signal of the light receiver 51, 52.

Alternatively, the light input threshold value may be set during a calibration mode, for example when the feeder unit 2 is removed and nothing is obstructing the light beam L1, L2. In this way, age degradation and/or small variations from factory specifications of the light emitter 41, 42 and/or the light receiver 51, 52 can be compensated for.

The light output A may also be decreased, for example when the light output A is higher than expected. This may occur after cleaning of the pollution or when the specifications of the respective light emitter 41, 42 are not in conformity with the factory specifications of said light emitter 41, 42. Hence, the light output A may be adjusted or corrected, both positively and negatively, to make sure that the respective light receiver 51, 52 can function in its optimal operating range or window.

The control unit 7, in the dynamic mode M1, is configured for increasing the light output A of the light emitter 41, 42 of the at least one detection pair 41, 51; 42, 52 up to an output threshold. The output threshold is less than ninety percent of a maximum output of the light emitter 41, 42 of the at least one detection pair 41, 51; 42, 52. The maximum output may be given as a factory specification or may be tested in the field.

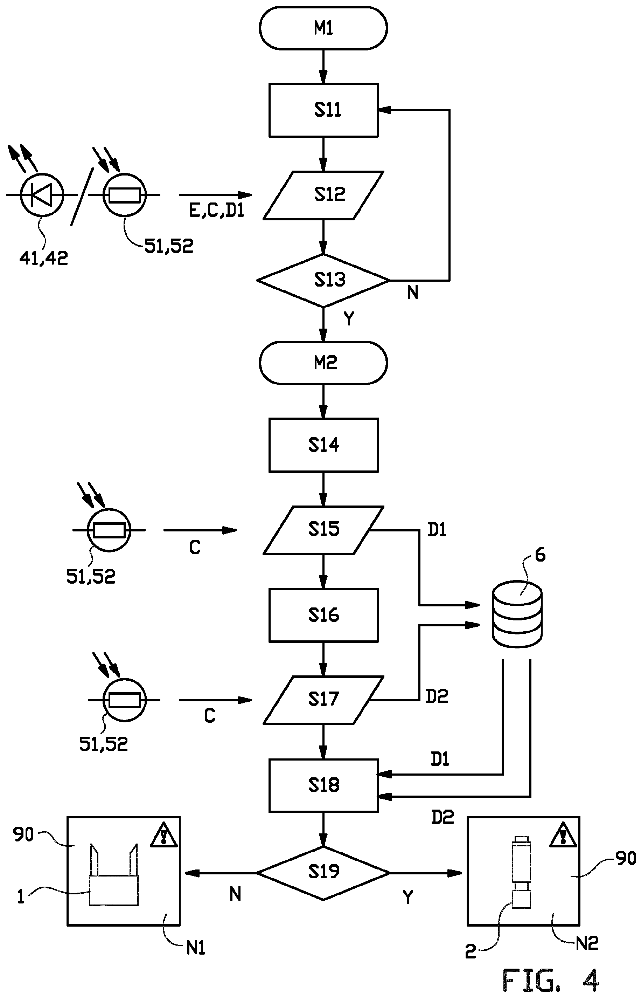

Figure 4 shows the steps of the method when the control unit 7 is operating in a test mode M2 or switched from the dynamic mode M1 to said test mode M2.

The steps of the method of figure 4 may overlap with or be executed in parallel to the steps of the method of figure 3. In particular, step S11 corresponds to step S1 which is the start of the dynamic mode M1. In one scenario, the next step (step S12) is also the same as step S2 of the previously discussed method in that the light input signal C or last light input value D1 is received at or processed by the control unit 7. In an alternative not being part of the claimed invention, step S12 can be based on the light output value E of one of the light emitters 41, 42.

In the next step, it is determined whether the last light input value D1 is equal to or below an input threshold or whether the light output value E is equal to or above the aforementioned output threshold. If the answer is 'yes' (Y) the control unit 7 switches from the dynamic mode M1 to the test mode M2. If the answer is 'no' (N), the dynamic mode M1 continues as shown in figure 3.

At the start of the test mode M2 (step S14), the control unit 7 is configured for receiving a first light input value D1 in a first situation in which the feeder unit 2 is received at the feeder dock 1 (step S15), as shown in figure **2****.** If this is already the case, then no separate action is required. If this is not the case, the control unit 7 may send control signals to an automated member, for example a robot, to place the feeder unit 2 on the feeder dock 1, or it may alert the pharmacist to place the feeder unit 2 on the feeder dock **1.** The method may involve additional checks (not shown) to check whether the feeder unit 2 has indeed been docked to or is present at the feeder dock 1. The first light input value D1 is stored in the memory 6.

Subsequently, the control unit 7 sends control signals to the automated member or instructions to the pharmacist to remove the feeder unit 2 from the feeder dock 1, to obtain the situation of figure 1 (step S16). The method may involve additional checks (not shown) to check whether the feeder unit 2 has indeed been removed or is absent from the feeder dock 1.

The control unit 7 is subsequently configured for receiving a second light input value D2 in a second situation in which the feeder unit 2 is removed from the feeder dock 1 (step S17). The second light input value D2 is also stored in the memory 6.

In the next step (step S18), the first light input values D1 representative of the first situation (figure 2), and the second light input value D2, representative of the second situation (figure 1), are compared to subsequently determine (step S19) whether the comparison result in a difference between the first light input value D1 and the second light input value D2. In particular, it is determined whether one of the following conditions is true:
a) one of the first situation and the second situation results in the light input values D1, D2 being different than in the other of the first situation and the second situation; or
b) the light input values D1, D2 are the same or within a predetermined tolerance of each other for the first situation and the second situation.

The predetermined tolerance may for example be less than ten percent of the first light input value D1 or less than five percent.

In particular, it is determined whether in condition a) the second light input value D2 is higher than the first light input value D1, which would be an indication that pollution of the feeder unit 2 is blocking or diffusing at least a part of the light beam L1, L2.

If condition a) is true, then the answer of step S19 is 'yes' (Y) and it is assumed that the change in light input value D1, D2 between the two situations is caused by the removal of the feeder unit 2. Consequently, the feeder unit 2 must be polluted. The control unit 7 is operationally connected to the alerting device 90 for sending a first notification N1 to the pharmacist via said alerting device 90. The first notification N1 may be indicative of a polluted state of the feeder unit 2.

If condition b) is true, then the answer of step s19 is 'no' (N) and it is assumed that the feeder dock 1 is polluted. The feeder unit 2 may also be polluted, but it is the feeder dock 1 that is so polluted that removing the feeder unit 2 does not result in a noticeable change in the light input values D1, D2. The control unit 7 sends a second notification N2 to the pharmacist via said alerting device 90. The second notification N2 may be indicative of a polluted state of the feeder dock 1.

It is to be understood that the above description is included to illustrate the operation of the preferred embodiments and is not meant to limit the scope of the invention. From the above discussion, many variations will be apparent to one skilled in the art, though the scope of protection is defined by the claims.

### LIST OF REFERENCE NUMERALS

- 1: feeder dock
- 10: base
- 11: dispensing drive
- 12: positioning member
- 14: outlet channel
- 2: feeder unit
- 20: transparent housing
- 21: supply chamber
- 22: dispensing section
- 23: dispensing mechanism
- 24: outlet
- 3: sensor system
- 41: first light emitter
- 42: second light emitter
- 51: first light receiver
- 52: second light receiver
- 6: memory
- 7: control unit
- 8: processor
- 9: assembly
- 90: alerting device
- A: light output
- B: light input
- C: light input signals
- D: light input values
- E: light output value
- L1: first light beam
- L2: second light beam
- M1: dynamic mode
- M2: test mode
- N1: first notification
- N2: second notification
- S1: step "start of dynamic mode"
- S2: step "receipt of light input signal"
- S3: step "has the light input signal been received
- S4: between dispensing intervals?" step "storing last light input value in the memory"
- S5: step "comparing last light input value with previously stored light input value(s)"
- S6: step "has the last light input value decreased compared to the previously stored light input value(s)?"
- S7: step "increase light output"
- S11: step "start of dynamic mode"
- S12: step "receipt of light output value or light input value"
- S13: step "is the light output value equal to or above an output threshold or is the light input value equal to or below an input threshold?"
- S14: step "start of test mode"
- S15: step "receiving the light input value in a first situation in which the feeder unit is received at the feeder dock"
- S16: step "removing the feeder unit from the feeder dock"
- S17: step "receiving the light input value in a second situation in which the feeder unit is removed from the feeder dock"
- S18: step "comparing light input values stored for the first situation and the second situation"
- S19: step "does the comparison result in a difference between the light input values stored for the first situation and the second situation?"

## Claims

1. Assembly (9) of a sensor system (3) for detecting events in an automatic dispensing process for dispensing discrete medicaments and a feeder dock (1) for docking a feeder unit (2), wherein a light emitter (41, 42) and/or a light receiver (51, 52) are provided in, on or at the feeder dock (1,)
wherein the feeder unit (2) comprises a dispensing mechanism (23) for dispensing the discrete medicaments one-by-one, wherein the feeder dock (1) comprises a dispensing drive (11) that is arranged for engaging with the dispensing mechanism (23) when said feeder unit (2) is received at the feeder dock (1),
wherein the feeder unit (2) has an at least partially transparent housing (20), wherein the light emitter (41, 42) and the light receiver (51, 52) are positioned such that, when the feeder unit (2) is received in the feeder dock (1), a light beam (L1, L2) travelling between the light emitter (41, 42) and the light receiver (51, 52) travels through a transparent section of the at least partially transparent housing (20) at least once,
wherein the sensor system (3) comprises the light emitter (41, 42) for generating light output (A), the light receiver (51, 52) for converting light input (B) into one or more light input signals (C) indicative of light input values (D1, D2, ..., Dn) and a control unit (7) that is operationally connected to said light emitter (41, 42) and said light receiver (51, 52), wherein the light emitter (41, 42) forms a detection pair (41, 51; 42, 52) with the light receiver (51, 52),
wherein the control unit (7) is configured for receiving the one or more light input signals (C) from the light receiver (51,52) and for operating in a dynamic mode (M1) in which the light output (A) of the light emitter (41, 42) is adjusted in response to the light input values (D1, D2, ..., Dn), wherein the light output (A) of the light emitter (41, 42) increases when the light input values (D1, D2, ..., Dn) drop below an input threshold, and wherein the input threshold is a calibration value that is determined based on one or more of the light input values (D1, D2, ..., Dn) during a calibration mode,
wherein the control unit (7) is operationally connected to the dispensing drive (11) to operate the dispensing mechanism (23) at dispensing intervals,
wherein the control unit (7), in the dynamic mode (M1), is configured for storing the light input values (D1, D2, ..., Dn) for the one or more light input signals (C) that are received between the dispensing intervals,
wherein the control unit (7) is configured for switching between the dynamic mode (M1) and a calibration mode,
wherein the control unit (7), in the calibration mode, is configured for storing one or more of the light input values (D1, D2, ..., Dn) as a calibration value when the feeder unit (2) is absent from the feeder dock (1),
wherein the control unit (7) is configured for switching between the dynamic mode (M1) and a test mode (M2), wherein the control unit (7), in the test mode (M2), is configured for storing one or more of the light input values (D1, D2, ..., Dn) in a first situation in which the feeder unit (2) is docked at the feeder dock (1) and for storing one or more of the light input values (D1, D2, ..., Dn) in a second situation in which the feeder unit (2) is removed from the feeder dock (1), and
wherein the control unit (7) is configured for switching from the dynamic mode (M1) to the test mode (M2) when the light output (A) of the light emitter (41, 42) is equal to or higher than an output threshold.

2. Assembly (9) according to claim 1, wherein the control unit (7), in the dynamic mode (M1), is configured for increasing the light output (A) of the light emitter (41, 42) when the light input values (D1, D2, ..., Dn) decrease over time.

3. Assembly (9) according to claim 1 or 2, wherein the input threshold is a predetermined input threshold value stored in a memory (6), and/or wherein the input threshold is equal to or less than sixty percent of a maximum light input signal of the light receiver (51, 52).

4. Assembly (9) according to any one of the preceding claims, wherein the control unit (7), in the dynamic mode (M1), is configured for storing the light input values (D1, D2, ..., Dn) continuously or at a regular interval, and/or is configured for increasing the light output (A) of the light emitter (41, 42) up to an output threshold, preferably wherein the output threshold is less than or equal to ninety percent of a maximum output of the light emitter (41, 42).

5. Assembly (9) according to any of the preceding claims, wherein the control unit (7) is further configured for comparing the one or more light input values (D1, D2, ..., Dn) stored in the first situation with the one or more light input values (D1, D2, ..., Dn) stored in the second situation.

6. Assembly (9) according to claim 5, wherein the control unit (7) is further configured for determining, based on the comparison, if one of the following conditions is true:
a) one of the first situation and the second situation results in the light input values (D1, D2, ..., Dn) being different than in the other of the first situation and the second situation; or
b) the light input values (D1, D2, ..., Dn) are the same or within a predetermined tolerance of each other for the first situation and the second situation.

7. Assembly (9) according to claim 6, wherein the assembly (9) comprises an alerting device (90) for notifying an operator, wherein the control unit (7) is operationally connected to the alerting device (90) for sending a first notification (N1) to the operator via said alerting device (90) when condition a) is true and for sending a second notification (N2) to the operator via said alerting device (90) when condition b) is true, preferably wherein the output threshold is less than ninety percent of a maximum output of the light emitter (41, 42).

8. Method for detecting events in an automatic dispensing process for dispensing discrete medicaments using the assembly of claim 1, wherein the method comprises the steps of:
- emitting light output (A) from the light emitter (41, 42) of the detection pair;
- receiving light input (B) by the light receiver (51, 52) of the detection pair and converting the light input (B) into one or more light input signals (C) indicative of light input values (D1, D2, ..., Dn);
- monitoring the light input values (D1, D2, ..., Dn) over time; and
- adjusting the light output (A) of the light emitter (41, 42) in response to the light input values (D1, D2, ..., Dn), wherein the light output (A) of the light emitter (41, 42) is increased when the light input values (D1, D2, ..., Dn) drop below an input threshold, and wherein the input threshold is a calibration value that is determined based on one or more of the light input values (D1, D2, ..., Dn) during a calibration mode.

9. Method according to claim 8, wherein the light output (A) of the light emitter (41, 42) is increased when the light input values (D1, D2, ..., Dn) decrease over time.

10. Method according to claim 8 or 9, wherein the input threshold is a predetermined input threshold value.

11. Method according to any one of claims 8 to 10, wherein the calibration value is determined when a light beam (L1, L2) between the light emitter (41, 42) and the light receiver (51, 52) is unobstructed.

12. Method according to any one of claims 8-11, wherein the input threshold is equal to or less than sixty percent of a maximum light input signal of the light receiver (51, 52).

13. Method according to any one of claims 8-12, wherein the light input values (D1, D2, ..., Dn) are monitored continuously or at a regular interval.

14. Method according to any one of claims 8-13, wherein the light output (A) of the light emitter (41, 42) is increased up to an output threshold, preferably wherein the output threshold is less than ninety percent of a maximum output of the light emitter (41, 42).

15. Method according to any one of claims 8-14, wherein the method further comprises the step of:
- dispensing the discrete medicaments one-by-one at dispensing intervals, wherein the light input values (D1, D2, ..., Dn) are monitored for the one or more light input signals (C) that are received between the dispensing intervals.

16. Method according to any one of claims 8-15, wherein the method further comprises the steps of: - providing a feeder dock (1) for docking a feeder unit (2), wherein the light emitter (41, 42) and/or the light receiver (51, 52) are provided in, on or at the feeder dock (1);
- in a first situation docking the feeder unit (2) at the feeder dock (1) and monitoring one or more of the light input values (D1, D2, ..., Dn) when the feeder unit (2) is docked at the feeder dock (1);
- in a second situation removing the feeder unit (2) from the feeder dock (1) and monitoring one or more of the light input values (D1, D2, ..., Dn) when the feeder unit (2) is removed from the feeder dock (1); and
- comparing the one or more light input values (D1, D2, ..., Dn) monitored in the first situation with the one or more light input values (D1, D2, ..., Dn) monitored in the second situation.

17. Method according to claim 16, wherein the method further comprises the step of:
- determining, based on the comparison, if one of the following conditions is true:
a) one of the first situation and the second situation results in the light input values (D1, D2, ..., Dn) being different than in the other of the first situation and the second situation; or
b) the light input values (D1, D2, ..., Dn) are the same or within a predetermined tolerance of each other for the first situation and the second situation, wherein the method further comprises the steps of:
- sending a first notification (N1) to an operator when condition a) is true; and
- sending a second notification (N2) to the operator when condition b) is true, preferably wherein the monitoring of the light input values (D1, D2, ..., Dn) in the first situation and the second situation and the comparison thereof are performed when the light output (A) of the light emitter (41, 42) is equal to or higher than an output threshold, more preferably wherein the output threshold is less than ninety percent of a maximum output of the light emitter (41, 42).

18. Computer program product comprising a non-transitory computer-readable medium (6) holding instructions that, when executed by a processor (8), cause the assembly (9) according to any one of claims 1-7 to perform the steps of the method according to any one of claims 8-17.

## Patentansprüche

1. Aufbau (9) eines Sensorsystems (3) zum Erfassen von Ereignissen in einem automatischen Abgabevorgang zum Abgeben von diskreten Arzneimitteln und einem Zuführdock (1) zum Andocken einer Zuführeinheit (2), wobei ein Lichtsender (41, 42) und/oder ein Lichtempfänger (51, 52) in, auf oder an dem Zuführdock (1,) vorgesehen ist,
wobei die Zuführeinheit (2) einen Abgabemechanismus (23) zum einzelnen Abgeben der diskreten Arzneimittel umfasst, wobei das Zuführdock (1) einen Abgabeantrieb (11) aufweist, der so angeordnet ist, dass er mit dem Abgabemechanismus (23) zusammenpasst, wenn die Zuführeinheit (2) im Zuführdock (1) aufgenommen ist,
wobei die Zuführeinheit (2) ein zumindest teilweise transparentes Gehäuse (20) aufweist, wobei der Lichtsender (41,42) und der Lichtempfänger (51, 52) so positioniert sind, dass, wenn die Zuführeinheit (2) im Zuführdock (1) aufgenommen ist, ein Lichtstrahl (L1, L2), der zwischen dem Lichtsender (41,42) und dem Lichtempfänger (51,52) verläuft, mindestens einmal durch einen transparenten Abschnitt des zumindest teilweise transparenten Gehäuses (20) verläuft,
wobei das Sensorsystem (3) den Lichtsender (41,42) zur Erzeugung der Lichtausgabe (A), den Lichtempfänger (51, 52) zur Umwandlung des Lichteingangs (B) in ein oder mehrere Lichteingangssignale (C), die auf Lichteingangswerte (D1, D2, ..., Dn) hinweisen, und eine Steuereinheit (7) umfasst, die betrieblich mit besagtem Lichtsender (41,42) und besagtem Lichtempfänger (51, 52) verbunden ist,
wobei der Lichtsender (41,42) ein Detektionspaar (41, 51; 42,52) mit dem Lichtempfänger (51, 52) bildet,
wobei die Steuereinheit (7) eingerichtet ist, die eines oder mehreren Lichteingangssignale (C) vom Lichtempfänger (51, 52) zu empfangen und im dynamischen Modus (M1) zu arbeiten, in welchem die Lichtausgabe (A) des Lichtsenders (41,42) als Reaktion auf die Lichteingangswerte (D1, D2,..., Dn) angepasst wird, wobei die Lichtausgabe (A) ansteigt, sobald die Lichteingangswerte (D1, D2,..., Dn) unter einen Eingangsschwellenwert fallen, und wobei der Eingangsschwellenwert ein Kalibrierwert ist, der basierend auf einem oder mehreren Lichteingangswerten (D1, D2,..., Dn) während eines Kalibriermodus bestimmt wird,
wobei die Steuereinheit (7) betrieblich mit dem Abgabeantrieb (11) verbunden ist, um den Abgabemechanismus (23) in Abgabeintervallen zu betreiben,
wobei die Steuereinheit (7) im dynamischen Modus (M1) eingerichtet ist, die Lichteingangswerte (D1, D2, ..., Dn) zu speichern für die eines oder mehreren Lichteingangssignale (C), die zwischen den Abgabeintervallen empfangen werden,
wobei die Steuereinheit (7) eingerichtet ist, zwischen dem dynamischen Modus (M1) und einem Kalibriermodus zu wechseln,
wobei die Steuereinheit (7) eingerichtet ist, im Kalibriermodus einen oder mehrere der Lichteingangswerte (D1, D2, ..., Dn) als Kalibrierwert zu speichern, wenn die Zuführeinheit (2) von dem Zuführdock (1) abwesend ist,
wobei die Steuereinheit (7) eingerichtet ist, zwischen dem dynamischen Modus (M1) und einem Testmodus (M2) zu wechseln,
wobei die Steuereinheit (7) eingerichtet ist, im Testmodus (M2) einen oder mehrere der Lichteingangswerte (D1, D2, ..., Dn) in einer ersten Situation zu speichern, in der die Zuführeinheit (2) am Zuführdock (1) angedockt ist, und einen oder mehrere der Lichteingangswerte (D1, D2,..., Dn) in einer zweiten Situation zu speichern, in der die Zuführeinheit (2) vom Zuführdock (1) entfernt ist, und
wobei die Steuereinheit (7) eingerichtet ist, vom dynamischen Modus (M1) in den Testmodus (M2) zu wechseln, wenn die Lichtausgabe (A) des Lichtsenders (41, 42) gleich oder höher als ein Ausgangsschwellenwert ist.

2. Aufbau (9) nach Anspruch 1, wobei die Steuereinheit (7), im dynamischen Modus (M1), eingerichtet ist, die Lichtausgabe (A) des Lichtsenders (41,42) zu erhöhen, wenn die Lichteingangswerte (D1, D2, ..., Dn) über die Zeit abnehmen.

3. Aufbau (9) nach Anspruch 1 oder 2, wobei der Eingangsschwellenwert ein vordefinierter Schwellenwert ist, der in einem Speicher (6) abgelegt ist, und/oder wobei der Eingangsschwellenwert gleich oder kleiner als sechzig Prozent eines maximalen Lichteingangssignals des Lichtempfängers (51, 52) ist.

4. Aufbau (9) nach einem der vorangehenden Ansprüche, wobei die Steuereinheit (7), im dynamischen Modus (M1), eingerichtet ist, die Lichteingangswerte (D1, D2, ..., Dn) kontinuierlich oder in regelmäßigen Intervallen zu speichern, und/oder die Lichtausgabe (A) des Lichtsenders (41, 42) bis zu einem Ausgangsschwellenwert zu erhöhen, vorzugsweise wobei der Ausgangsschwellenwert weniger als oder gleich neunzig Prozent einer maximalen Ausgabe des Lichtsenders (41, 42) ist.

5. Aufbau (9) nach einem der vorangehenden Ansprüche, wobei die Steuereinheit (7) ferner eingerichtet ist, die in der ersten Situation gespeicherten Lichteingangswerte (D1, D2, ..., Dn) mit den in der zweiten Situation gespeicherten Lichteingangswerten (D1, D2, ..., Dn) zu vergleichen.

6. Aufbau (9) nach Anspruch 5, wobei die Steuereinheit (7) ferner eingerichtet ist, auf Basis des Vergleichs zu bestimmen, ob eine der folgenden Bedingungen zutrifft:
a) in einer der ersten Situation und der zweiten Situation führen die Lichteingangswerte (D1, D2,..., Dn) zu einem anderen Wert als in der jeweils anderen Situation; oder
b) die Lichteingangswerte (D1, D2,..., Dn) sind in der ersten und zweiten Situation gleich oder liegen innerhalb einer vorbestimmten Toleranz zueinander.

7. Aufbau (9) nach Anspruch 6, worin die Aufbau (9) eine Alarmvorrichtung (90) zum Benachrichtigen eines Bedieners umfasst, wobei die Steuereinheit (7) betrieblich mit der Alarmvorrichtung (90) verbunden ist, um eine erste Benachrichtigung (N1) an den Bediener über besagte Alarmvorrichtung (90) zu senden, wenn Bedingung a) zutrifft, und eine zweite Benachrichtigung (N2) an den Bediener über besagte Alarmvorrichtung (90) zu senden, wenn Bedingung b) zutrifft, vorzugsweise wobei der Ausgangsschwellenwert weniger als neunzig Prozent einer maximalen Ausgabe des Lichtsenders (41, 42) ist.

8. Verfahren zum Erfassen von Ereignissen in einem automatischen Abgabevorgang zur Abgabe von diskreten Arzneimitteln unter Verwendung der Aufbau (9) nach Anspruch 1, wobei das Verfahren die folgenden Schritte umfasst:
- Lichtausgabe (A) vom Lichtsender (41, 42) des Detektionspaares;
- Lichtempfang (B) durch den Lichtempfänger (51, 52) des Detektionspaares und Umwandeln des Lichteingangs (B) in eines oder mehrere Lichteingangssignale (C), die auf Lichteingangswerte (D1, D2, ..., Dn) hinweisen;
- Überwachen der Lichteingangswerte (D1, D2, ..., Dn) über die Zeit; und
- Anpassen der Lichtausgabe (A) des Lichtsenders (41, 42) als Reaktion auf die Lichteingangswerte (D1, D2, ..., Dn), wobei die Lichtausgabe (A) erhöht wird, wenn die Lichteingangswerte (D1, D2, ..., Dn) unter einen Eingangsschwellenwert fallen, und wobei der Eingangsschwellenwert ein Kalibrierwert ist, der basierend auf einem oder mehreren Lichteingangswerten (D1, D2, ..., Dn) während eines Kalibriermodus bestimmt wird.

9. Verfahren nach Anspruch 8, wobei die Lichtausgabe (A) des Lichtsenders (41, 42) erhöht wird, wenn die Lichteingangswerte (D1, D2, ..., Dn) im Zeitverlauf abnehmen.

10. Verfahren nach Anspruch 8 oder 9, wobei der Eingangsschwellenwert ein vordefinierter Schwellenwert ist.

11. Verfahren nach einem der Ansprüche 8 bis 10, wobei der Kalibrierwert bestimmt wird, wenn ein Lichtstrahl (L1, L2) zwischen Lichtsender (41, 42) und Lichtempfänger (51, 52) ungehindert ist.

12. Verfahren nach einem der Ansprüche 8 bis 11, wobei der Eingangsschwellenwert gleich oder kleiner als sechzig Prozent eines maximalen Lichteingangssignals des Lichtempfängers (51, 52) ist.

13. Verfahren nach einem der Ansprüche 8 bis 12, wobei die Lichteingangswerte (D1, D2, ..., Dn) kontinuierlich oder in regelmäßigen Intervallen überwacht werden.

14. Verfahren nach einem der Ansprüche 8 bis 13, wobei die Lichtausgabe (A) des Lichtsenders (41,42) bis zu einem Ausgangsschwellenwert erhöht wird, vorzugsweise wobei der Ausgangsschwellenwert weniger als neunzig Prozent einer maximalen Ausgabe des Lichtsenders (41, 42) ist.

15. Verfahren nach einem der Ansprüche 8 bis 14, wobei das Verfahren ferner den Schritt umfasst des:
- einzelnen Abgebens der diskreten Arzneimittel in Abgabeintervallen, wobei die Lichteingangswerte (D1, D2, ..., Dn) für das eine oder die mehreren Lichteingangssignale (C), die zwischen den Abgabeintervallen empfangen werden, überwacht werden.

16. Verfahren nach einem der Ansprüche 8 bis 15, wobei das Verfahren ferner folgende Schritte umfasst:
- Bereitstellen eines Zuführdocks (1) zum Andocken einer Zuführeinheit (2), wobei ein Lichtsender (41,42) und/oder ein Lichtempfänger (51, 52) in, auf oder an dem Zuführdock (1) vorgesehen ist;
- in einer ersten Situation, Andocken der Zuführeinheit (2) am Zuführdock (1) und Überwachen eines oder mehrerer Lichteingangswerte (D1, D2, ..., Dn), wenn die Zuführeinheit (2) am Zuführdock (1) angedockt ist;
- in einer zweiten Situation, Entfernen der Zuführeinheit (2) vom Zuführdock (1) und Überwachen eines oder mehrerer Lichteingangswerte (D1, D2, ..., Dn), wenn die Zuführeinheit (2) vom Zuführdock (1) entfernt ist; und
- Vergleichen des einen oder der mehreren überwachten Lichteingangswerte (D1, D2, ..., Dn) in der ersten Situation mit denen in der zweiten Situation.

17. Verfahren nach Anspruch 16, wobei das Verfahren ferner umfasst:
- Bestimmen, basierend auf dem Vergleich, ob eine der folgenden Bedingungen zutrifft:
a) in einer der ersten Situation und der zweiten Situation führen die Lichteingangswerte (D1, D2, ..., Dn) zu unterschiedlichen Werten; oder
b) die Lichteingangswerte (D1, D2, ..., Dn) sind gleich oder innerhalb einer vorbestimmten Toleranz zueinander, wobei das Verfahren ferner folgende Schritte umfasst:
- Senden einer ersten Benachrichtigung (N1) an einen Bediener, wenn Bedingung a) zutrifft; und
- Senden einer zweiten Benachrichtigung (N2) an den Bediener, wenn Bedingung b) zutrifft, vorzugsweise wobei die Überwachung der Lichteingangswerte (D1, D2, ..., Dn) in der ersten Situation und der zweiten Situation sowie deren Vergleich nur durchgeführt werden, wenn die Lichtausgabe (A) des Lichtsenders (41,42) gleich oder höher als ein Ausgangsschwellenwert ist, stärker bevorzugt, wenn der Ausgangsschwellenwert weniger als neunzig Prozent einer maximalen Ausgabe des Lichtsenders (41, 42) ist.

18. Computerprogrammprodukt, umfassend ein nichtflüchtiges computerlesbares Medium (6), das Anweisungen enthält, die, wenn von einem Prozessor (8) ausgeführt, die Aufbau (9) gemäß einem der Ansprüche 1-7 veranlassen, die Schritte des Verfahrens gemäß einem der Ansprüche 8-17 durchzuführen.

## Revendications

1. Ensemble (9) d'un système de capteurs (3) pour la détection d'événements dans un processus de distribution automatique pour distribuer des médicaments discrets et d'une station d'accueil d'alimentation (1) pour l'accueil d'une unité d'alimentation (2), où un émetteur de lumière (41, 42) et/ou un récepteur de lumière (51, 52) sont disposés dans, sur ou au niveau de la station d'accueil d'alimentation (1),
où l'unité d'alimentation (2) comprend un mécanisme de distribution (23) pour distribuer les médicaments discrets un par un, où la station d'accueil d'alimentation (1) comprend un entraînement de distribution (11) qui est conçu pour s'engager avec le mécanisme de distribution (23) lorsque ladite unité d'alimentation (2) est reçue au niveau de la station d'accueil d'alimentation (1),
où l'unité d'alimentation (2) a un boîtier (20) au moins partiellement transparent, où l'émetteur de lumière (41, 42) et le récepteur de lumière (51, 52) sont positionnés de telle sorte que, lorsque l'unité d'alimentation (2) est reçue dans la station d'accueil d'alimentation (1), un faisceau de lumière (L1, L2) se déplaçant entre l'émetteur de lumière (41, 42) et le récepteur de lumière (51, 52) traverse au moins une fois une section transparente dudit boîtier (20) au moins partiellement transparent,
où le système de capteurs (3) comprend l'émetteur de lumière (41, 42) pour générer une sortie de lumière (A), le récepteur de lumière (51, 52) pour convertir l'entrée de lumière (B) en un ou plusieurs signaux d'entrée de lumière (C) indiquant des valeurs d'entrée de lumière (D1, D2, ..., Dn), et une unité de commande (7) qui est connectée de manière opérationnelle audit émetteur de lumière (41, 42) et audit récepteur de lumière (51, 52), où l'émetteur de lumière (41, 42) forme avec le récepteur de lumière (51, 52) une paire de détection (41, 51 ; 42, 52),
où l'unité de commande (7) est configurée pour recevoir l'un ou plusieurs signaux d'entrée de lumière (C) du récepteur de lumière (51, 52) et pour fonctionner dans un mode dynamique (M1) dans lequel la sortie de lumière (A) de l'émetteur de lumière (41, 42) est ajustée en réponse aux valeurs d'entrée de lumière (D1, D2, ..., Dn), où la sortie de lumière (A) de l'émetteur de lumière (41, 42) augmente lorsque les valeurs d'entrée de lumière (D1, D2, ..., Dn) tombent en dessous d'un seuil d'entrée, et où le seuil d'entrée est une valeur d'étalonnage qui est déterminée sur la base d'une ou plusieurs des valeurs d'entrée de lumière (D1, D2, ..., Dn) pendant un mode d'étalonnage,
où l'unité de commande (7) est connectée de manière opérationnelle à l'entraînement de distribution (11) pour faire fonctionner le mécanisme de distribution (23) à des intervalles de distribution,
où l'unité de commande (7), dans le mode dynamique (M1), est configurée pour stocker les valeurs d'entrée de lumière (D1, D2, ..., Dn) pour l'un ou plusieurs signaux d'entrée de lumière (C) qui sont reçus entre les intervalles de distribution,
où l'unité de commande (7) est configurée pour commuter entre le mode dynamique (M1) et un mode d'étalonnage,
où l'unité de commande (7), dans le mode d'étalonnage, est configurée pour stocker une ou plusieurs des valeurs d'entrée de lumière (D1, D2, ..., Dn) comme une valeur d'étalonnage lorsque l'unité d'alimentation (2) est absente de la station d'accueil d'alimentation (1),
où l'unité de commande (7) est configurée pour commuter entre le mode dynamique (M1) et un mode test (M2), où l'unité de commande (7), dans le mode test (M2), est configurée pour stocker une ou plusieurs des valeurs d'entrée de lumière (D1, D2, ..., Dn) dans une première situation dans laquelle l'unité d'alimentation (2) est accueillie dans la station d'accueil d'alimentation (1), et pour stocker une ou plusieurs des valeurs d'entrée de lumière (D1, D2, ..., Dn) dans une deuxième situation dans laquelle l'unité d'alimentation (2) est retirée de la station d'accueil d'alimentation (1), et
où l'unité de commande (7) est configurée pour commuter du mode dynamique (M1) au mode test (M2) lorsque la sortie de lumière (A) de l'émetteur de lumière (41, 42) est égale ou supérieure à un seuil de sortie.

2. Ensemble (9) selon la revendication 1, où l'unité de commande (7), dans le mode dynamique (M1), est configurée pour augmenter la sortie de lumière (A) de l'émetteur de lumière (41, 42) lorsque les valeurs d'entrée de lumière (D1, D2, ..., Dn) diminuent au fil du temps.

3. Ensemble (9) selon la revendication 1 ou 2, où le seuil d'entrée est une valeur seuil d'entrée prédéterminée stockée dans une mémoire (6), et/ou où le seuil d'entrée est égal ou inférieur à soixante pour cent d'un signal d'entrée de lumière maximal du récepteur de lumière (51, 52).

4. Ensemble (9) selon l'une quelconque des revendications précédentes, où l'unité de commande (7), en mode dynamique (M1), est configurée pour stocker les valeurs d'entrée de lumière (D1, D2, ..., Dn) en continu ou à un intervalle régulier, et/ou est configurée pour augmenter la sortie de lumière (A) de l'émetteur de lumière (41, 42) jusqu'à un seuil de sortie, de préférence, où le seuil de sortie est inférieur ou égal à 90 pour cent d'une sortie maximale de l'émetteur de lumière (41, 42).

5. Ensemble (9) selon l'une quelconque des revendications précédentes, où l'unité de commande (7) est en outre configurée pour comparer l'une ou plusieurs valeurs d'entrée de lumière (D1, D2, ..., Dn) stockées dans la première situation avec l'une ou plusieurs valeurs d'entrée de lumière (D1, D2, ..., Dn) stockées dans la deuxième situation.

6. Ensemble (9) selon la revendication 5, où l'unité de commande (7) est en outre configurée pour déterminer, sur la base de la comparaison, si l'une des conditions suivantes est vraie :
a) l'une parmi la première situation et la deuxième situation entraîne des valeurs d'entrée de lumière (D1, D2, ..., Dn) différentes de celles de l'autre de la première situation et de la deuxième situation ; ou
b) les valeurs d'entrée de lumière (D1, D2, ..., Dn) sont identiques ou se situent dans une tolérance prédéterminée l'une de l'autre pour la première situation et la deuxième situation.

7. Ensemble (9) selon la revendication 6, où l'ensemble (9) comprend un dispositif d'alerte (90) pour notifier un opérateur, où l'unité de commande (7) est connectée de manière opérationnelle au dispositif d'alerte (90) pour envoyer une première notification (N1) à l'opérateur via ledit dispositif d'alerte (90) lorsque la condition a) est vraie et pour envoyer une deuxième notification (N2) à l'opérateur via ledit dispositif d'alerte (90) lorsque la condition b) est vraie, de préférence où le seuil de sortie est inférieur à 90 pour cent d'une sortie maximale de l'émetteur de lumière (41, 42).

8. Procédé de détection d'événements dans un processus de distribution automatique de médicaments discrets utilisant l'ensemble selon la revendication 1, où le procédé comprend les étapes de :
- émettre une sortie de lumière (A) à partir de l'émetteur de lumière (41, 42) de la paire de détection ;
- recevoir une entrée de lumière (B) par le récepteur de lumière (51, 52) de la paire de détection et convertir l'entrée de lumière (B) en un ou plusieurs signaux d'entrée de lumière (C) indiquant des valeurs d'entrée de lumière (D1, D2, ..., Dn) ;
- surveiller les valeurs d'entrée de lumière (D1, D2, ..., Dn) au fil du temps ; et
- ajuster la sortie de lumière (A) de l'émetteur de lumière (41, 42) en réponse aux valeurs d'entrée de lumière (D1, D2, ..., Dn), où la sortie de lumière (A) de l'émetteur de lumière (41, 42) est augmentée lorsque les valeurs d'entrée de lumière (D1, D2, ..., Dn) tombent en dessous d'un seuil d'entrée, et où le seuil d'entrée est une valeur d'étalonnage qui est déterminée sur la base d'une ou plusieurs des valeurs d'entrée de lumière (D1, D2, ..., Dn) pendant un mode d'étalonnage.

9. Procédé selon la revendication 8, où la sortie de lumière (A) de l'émetteur de lumière (41, 42) est augmentée lorsque les valeurs d'entrée de lumière (D1, D2, ..., Dn) diminuent au fil du temps.

10. Procédé selon la revendication 8 ou 9, où le seuil d'entrée est une valeur seuil d'entrée prédéterminée.

11. Procédé selon l'une quelconque des revendications 8 à 10, où la valeur d'étalonnage est déterminée lorsqu'un faisceau de lumière (L1, L2) entre l'émetteur de lumière (41, 42) et le récepteur de lumière (51, 52) est non obstrué.

12. Procédé selon l'une quelconque des revendications 8 à 11, où le seuil d'entrée est égal ou inférieur à soixante pour cent d'un signal d'entrée de lumière maximal du récepteur de lumière (51, 52).

13. Procédé selon l'une quelconque des revendications 8 à 12, où les valeurs d'entrée de lumière (D1, D2, ..., Dn) sont surveillées en continu ou à un intervalle régulier.

14. Procédé selon l'une quelconque des revendications 8 à 13, où la sortie de lumière (A) de l'émetteur de lumière (41, 42) est augmentée jusqu'à un seuil de sortie, de préférence où le seuil de sortie est inférieur à 90 pour cent d'une sortie maximale de l'émetteur de lumière (41, 42).

15. Procédé selon l'une quelconque des revendications 8 à 14, où le procédé comprend en outre l'étape de :
- distribuer les médicaments discrets un par un à des intervalles de distribution, où les valeurs d'entrée de lumière (D1, D2, ..., Dn) sont surveillées pour l'un ou plusieurs signaux d'entrée de lumière (C) qui sont reçus entre les intervalles de distribution.

16. Procédé selon l'une quelconque des revendications 8 à 15, où le procédé comprend en outre les étapes de : - fournir une station d'accueil d'alimentation (1) pour l'accueil d'une unité d'alimentation (2), où l'émetteur de lumière (41, 42) et/ou le récepteur de lumière (51, 52) sont disposés dans, sur ou au niveau de la station d'accueil d'alimentation (1) ;
- dans une première situation, accueillir l'unité d'alimentation (2) au niveau de la station d'accueil d'alimentation (1) et surveiller une ou plusieurs des valeurs d'entrée de lumière (D1, D2, ..., Dn) lorsque l'unité d'alimentation (2) est accueillie dans la station d'accueil d'alimentation (1) ;
- dans une deuxième situation, retirer l'unité d'alimentation (2) de la station d'accueil d'alimentation (1) et surveiller une ou plusieurs des valeurs d'entrée de lumière (D1, D2, ..., Dn) lorsque l'unité d'alimentation (2) est retirée de la station d'accueil d'alimentation (1) ; et
- comparer l'une ou plusieurs valeurs d'entrée de lumière (D1, D2, ..., Dn) surveillées dans la première situation avec l'une ou plusieurs valeurs d'entrée de lumière (D1, D2, ..., Dn) surveillées dans la deuxième situation.

17. Procédé selon la revendication 16, où le procédé comprend en outre l'étape de :
- déterminer, sur la base de la comparaison, si l'une des conditions suivantes est vraie :
a) l'une parmi la première situation et la deuxième situation entraîne des valeurs d'entrée de lumière (D1, D2, ..., Dn) différentes de celles de l'autre de la première situation et de la deuxième situation ; ou
b) les valeurs d'entrée de lumière (D1, D2, ..., Dn) sont identiques ou se situent dans une tolérance prédéterminée l'une de l'autre pour la première situation et la deuxième situation, où le procédé comprend en outre les étapes de :
- envoyer une première notification (N1) à un opérateur lorsque la condition a) est vraie ; et
- envoyer une deuxième notification (N2) à l'opérateur lorsque la condition b) est vraie, de préférence où la surveillance des valeurs d'entrée de lumière (D1, D2, ..., Dn) dans la première situation et dans la deuxième situation, et la comparaison de celles-ci sont effectuées lorsque la sortie de lumière (A) de l'émetteur de lumière (41, 42) est égale ou supérieure à un seuil de sortie, plus préférablement, où le seuil de sortie est inférieur à 90 pour cent d'une sortie maximale de l'émetteur de lumière (41, 42).

18. Produit programme d'ordinateur comprenant un support non transitoire lisible par ordinateur (6) contenant des instructions qui, lorsqu'elles sont exécutées par un processeur (8), amènent l'ensemble (9) selon l'une quelconque des revendications 1 à 7 à effectuer les étapes du procédé selon l'une quelconque des revendications 8 à 17.
